# EUROPEAN PATENT SPECIFICATION

(11) **EP 0 357 320 B1**
(45) Date of publication and mention of the grant of the patent: **20.04.1994**
(21) Application number: 89308500.1
(22) Date of filing: 22.08.1989
(51) Int. Cl.: A23K 1/00, A23L 1/28, C12N 1/22, A01G 1/04

(54) **Obtaining edible material from fungus-digested medium**
Erzeugung von Nahrungsmittel aus einem pilzgegärtem Medium
Fabrication de matière comestible à partir d'un milieu digéré par un champignon

(30) Priority: 30.08.1988 JP 217399/88
(43) Date of publication of application: 07.03.1990
(73) Proprietor: Ikeda, Hisakazu, Hanishina-gun Nagano 359-06 (JP)
(72) Inventor: Ikeda, Hisakazu, Hanishina-gun Nagano 359-06 (JP)
(74) Representative: Burford, Anthony Frederick

(56) References cited:
- WO-A-80/00400
- DE-A- 3 600 892
- GB-A- 2 074 558
- US-A- 4 711 787
- PATENT ABSTRACTS OF JAPAN, vol. 12, no. 491 (C-554)[3338], 21st December 1988; & JP-A-63 202 356 (HITOSHI NAGAOKA) 22-08-1988
- PATENT ABSTRACTS OF JAPAN, vol. 9, no. 309 (C-318)[2032], 5th December 1985; & JP-A-60 149 369 (HITOSHI NAGAOKA) 06-08-1985
- Lebensmittellexikon 1981, VEB, pages 744-747

## Description

This invention relates to an edible material obtained by processing a digested medium resulting from the cultivation of fungi. The material can be used as a food for animals and humans.

Main feed for domestic animals is cereals which are also food for humans. Grasses which can not be eaten by humans are generally used as feed for ruminants.

In order to reduce the cost of feed, husks such as rice husks and corncob are used partially as crude feed for ruminants, however, they have not been widely used because poor results are obtained and most of the husks are being disposed of.

Feeding tests have been conducted with a material obtained by decomposing, by the action of yeast, a waste medium resulting from the cultivation of mushrooms in a medium containing mainly sawdust.

However, the tests in most cases encountered large problems in continuous feeding. For example, in the case of feeding the above material to twenty cattle, very satisfactory growth was observed for 7 months, but,thereafter, the animals lost their appetites rapidly, exhibited robot-like actions by the stiffening of muscles and all of them died within one month.

The reason is not yet known, but it seems that resins, tar and cyanic compounds originating from the sawdust were probably accumulated in the animal's body and the problem appeared when the accumulation had increased beyond some level.

GB-A-2074558 (published 4th November 1981) discloses culturing hyphae of edible mushrooms on mixtures of peat with 10 to 40% by weight cereals and removing the fruit bodies after germination. The residue is used as a livestock feed or fermented to provide a fertilizer.

WO-A-8000400 (published 20th March 1980) discloses cultivating Pleurotus hyphae on a carbohydrate-containing food product to increase the protein content of the food product. Specified food products include lignin-rich grassy material and cereals.

The present inventor has carried out research for many years for cultivating mushrooms employing grassy materials avoiding woody materials such as sawdust.

It has been found that good results are obtained when the digested medium obtained from the above cultivation is improved in taste by employing the medium in a small amount and adding thereto the lees of soybean sauce or apple juice, molasses or compound feed. However, when the ratio of the medium to the additives is increased, the feed declined in taste and poor results are obtained.

Furthermore, in the case of employing a digested medium obtained from the cultivation of mushrooms as a feed, a long period of time is required to accustom animals to the feed and the conversion of feed requires complicated procedures. The digested medium is not suitable for human food because of its odor.

It is an object of the present invention to solve these problems. According to this invention, there is provided an edible material which comprises a digested medium obtained by cultivating a non-toxic fungus in a medium containing finely cut grassy material which is rich in lignin together with nutrient sources, having the specific odor of the medium removed therefrom.

As the grassy material is rich in lignin, there may be exemplified, rice husks, barley husks, corncob, bagasse, the husks of buck-wheat or soybean, and stems or leaves of Gramineae. These materials preferably are employed as a cut form having a volume density 0.15 to 0.66.

As the nutrient sources, it is preferable to employ materials such as rice bran, wheat bran, soybean-curd lees (residue left after pressing soybean milk out of boiled soybean). If desired, grain powder such as rice powder, wheat flour, or soybean powder may be employed.

When the nutrient sources are calculated as dry materials containing 10% of water, the ratio of the cut grassy material to the nutrient sources preferably is 3-4 : 2 by weight.

As the fungus to be cultured, any of edible mushroons which can be cultivated, such as Enokitake (Flammulina veltipes), Hiratake (Pleurocus ostreatus), Bunashimeji (Hypsizygus marmoreus), Maitake (Grifola frandose), Nameko (Pholiota nameko) and Shiitake (Lentinula edodes) may be employed.

The cultivation of mushrooms can be carried out by conventional methods employed in mushroom cultivation. For example, in the case of Enokitake, a medium is filled into bottles and sterilized by heating. Enokitake spores are inoculated on the medium and cultivated at 20°C for 20 to 30 days. The surface of the medium is then scraped to expose new surface. After allowing to stand at 10°C for 8 to 10 days, the fruit bodies of Enokitake growing in banks 2-3 cm in height are wrapped with paper to prevent them from toppling over and, after further several days, matured fruit bodies are harvested.

In the case of other mushrooms, their cultivation may be carried out depending on the kind of mushroom by conventional or known methods or methods similar thereto.

During cultivation, hyphae grow in the medium and the ingredients of the medium are digested and decomposed with enzymes secreted from the hyphae.

In the present invention, mushrooms are cultivated to digest their culture mediums and the harvest of fruit bodies is only of subsidiary importance. Therefore, as long as the medium is digested, the harvest of the fruit bodies my be omitted and any non-toxic fungus may be cultured.

The digested medium thus obtained has a specific odor like a decayed fruit body of a mushroom, and the odor is removed in the present invention.

The removal of the odor can be carried out by heating the digested medium.

The temperature of the heating is preferably not less than 70°C and, if desired, a temperature around 100°C or more may be employed.

Suitable time required for the heating depends on the heating temperature. For example, it is 1 to 2 hours at around 100°C.

The digested medium is sterilized simultaneously with deodorization by heating and therefore, it can be preserved, and further it can be dried by heating to make a dried material.

The heating may be carried out directly by fire or indirectly by atmospheric or high pressure steam.

The removal of odor from the digested medium also can be carried out by fermenting the medium.

In the fermentation, the digested medium may be employed as it is or after sterilization by heating.

The fermentation is carried out by fermenting the medium after addition of a non-toxic microorganism.

As the microorganism, there may be exemplified, a yeast belonging to Torula, Saccharomyces, Schizosaccharomyces, a fungus belonging to Aspergillus, Mucor, and a bacteria such as a lactic-acid bacteria belonging to Lactobacillus, Streptococcus or Bacillus, and Bacillus natto. Among them, particularly preferable is a yeast for making Sake (Japanese rice wine), beer or bread, Aspergillus oryzae, a lactic-acid bacteria and Bacillus natto.

Each of these microorganisms can be employed alone or as a mixture thereof.

The fermentation is carried out generally at around 20°C to 40°C. When sufficient heat is generated by the fermentation, there is no need to apply external heat.

If the fermentation is carried out sufficiently, the specific odor of the digested medium is removed and, in most cases, an aroma results from the fermentation.

When the digested medium deodorized as above is given to domestic animals, it can be almost completely ingested with a very high ingestion rate. Accordingly, a large amount of crude protein and other nutrient sources contained in the medium are utilized effectively. Therefore, employing it as a concentrated nutrient, the healthy fattening of warm-blooded animals such as domestic animals can be achieved. It is also suitable as a feed for cold-blooded animals such as fish. Furthermore, it can be used as a food for humans, because it is rich in nutrients and its bad odor has been removed. Excellent results have been obtained in fattening tests of domestic animals.

The present food material can be employed, if desired, in combination with other feed, bait or food, depending on the animal to be fed.

The deodorized digested medium may be used as it is, however, from the view point of handling, it is generally preferable to preserve it in a dried form and it may be processed to powder or pellets of the dried material.

Hereinafter, the present invention is further explained in the form of examples.

### Example 1

50 g of corncob, 40 g of rice husks and 30 g of the husks of buck-wheat were cut separately to 0.22 volume density and mixed together. To the mixture, 150 g of soybean-curd lees, 50 g of wheat bran and 10 g of rice bran were added as nutrient sources, and the water content of the mixture was adjusted to 63%. The adjusted mixture was filled into 800 ml bottles as culture medium whereon Hiratake was cultivated in conventional manner.

The fruit bodies of Hiratake thus grown were harvested and the residual digested medium was dried by heating in a drying room.

In the drying room (which had been converted from a vinyl house for drying grass) room temperature was elevated to about 80°C by direct irradiation of the sun. The digested medium was placed in nursery bed containers, spread in thin layers, and then the containers were placed on a draining board in the drying room, and heated by sun light in fine weather for 2 days.

The dried material thus obtained lost its bad odor and acquired an aroma like concentrated feed. The dried material was fed to pigs, sheep and cattle. The animals ate up the material willingly and there was no need to add other feed or tasty material.

### Example 2

Each 20 kg of the digested material obtained in the same manner as in Example 1 was filled into vinyl bags. The filled bags were placed on shelves in an atmospheric steam sterilizer without being piled upon each other and heated for 2 hours by steam and then allowed to stand for 1 hour.

The heat-treated, digested medium thus obtained acquired an aroma like boiled rice, and, when it was fed to pigs, sheep and cattle, the animals ate it up willingly.

### Example 3

150 kg of digested medium obtained in the same manner as in Example 1, 30 kg of molasses for feed (a feed product for cattle containing molasses; produced by Fuji Shiryo Co., Ltd.), 10 kg of rice bran and 6 kg of Vitakogen (a special feed containing lactic-acid bacteria, Bacillus subtilis, yeast, Aspergillus oryzae etc.; produced by Seiwa Sangyo Co., Ltd.) were mixed together, and the water content of the mixture was adjusted to 50%. Then the mixture was heaped on a floor and covered with a mat. It was summer and the temperature of the open air was 30°C. After 1 hour, the mixture began to ferment and after 10 hours the temperature of the mixture rose to 45°C and then gradually fell to 25°C after 20 hours.

The medium thus treated had a sweet and sour aroma and was filled in vinyl bags and stored.

### Example 4

A digested medium was prepared in the same manner as in Example 1, except that 80 g of corncob, 40 g of rich husks, 200 g of soybean-curd lees and 50 g of wheat husks were employed and the water content is adjusted to 64%.

On the medium, a mushroom was cultivated as described in Example 1 to give a digested medium. To 200 kg of the digested medium, 100 kg of a compound feed for piglets (Meiji Highmax, produced by Meiji Milk Products Co., Ltd., Toyko) and 5 kg of Biopremix (a preparation containing Bacillus natto, lactic-acid bacteria, yeast and Aspergillus oryzae; produced by Matsumoto Microbiology Research Co., Ltd., Matsumoto) were added and mixed together. 20 kg Portions of the mixture were filled into vinyl bags and subjected to fermentation. The atmospheric temperature was 27°C. After 3 days, the fermented material was suitable for feeding to piglets, and after 7 days it acquired a very sweet and sour aroma and showed increased taste to piglets.

### Example 5

An experiment was carried out in the same manner as Example 4, except for employing yoghurt instead of Biopremix, to obtain results similar to Example 4.

In the above Examples 3 to 5, small amounts of nutrient sources for the fermentation are required, however, the bad odor of the digested medium was replaced by the aroma of the fermentation and the medium had a very increased ingestion ratio.

## Claims

1. A method for producing an edible material which comprises cultivating a non-toxic fungus in a nutrient medium containing finely cut lignin-rich grassy material, recovering the medium digested by the cultivation and then removing the specific odor of the digested medium by heating or fermenting the medium.

2. A method as claimed in Claim 1, wherein the grassy material is selected from rice husks, barley husks, corncob, bagasse, buck-wheat husks, soybean husks the stems or leaves of Gramineae, and mixtures thereof.

3. A process as claimed in Claim 1 or Claim 2, wherein the cut grassy material has a volume density of 0.15 to 0.66.

4. A process as claimed in any one of the preceding claims, wherein the nutrient medium contains also a nutrient source selected from rice bran, wheat bran, soybean-curd lees, rice powder, wheat flour, soybean powder and mixtures thereof.

5. A process as claimed in any one of the preceding claims, wherein the weight ratio of grassy material to nutrient sources is 3:2 to 4:2.

6. A process as claimed in any one of the preceding claims, wherein the non-toxic fungus is an edible mushroom.

7. A method as claimed in any one of the preceding claims, wherein said heating is carried out at a temperature not less than 70°C.

8. A method as claimed in Claim 7, wherein said heating is carried out by steam at a temperature not less than 95°C.

9. A method as claimed in any one of Claims 1 to 6, wherein said fermentation is carried out using a non-toxic microorganism selected from yeasts, fungi and bacteria.

10. A method as claimed in Claim 9, wherein the microorganism is a yeast, Lactobacillus, Aspergillus or Bacillus natto.

11. An edible material which comprises a digested medium obtained by cultivating a non-toxic fungus in a nutrient medium containing finely cut lignin-rich grassy material and having the specific odor of said digested medium removed therefrom by heating or fermenting the medium.

## Patentansprüche

1. Verfahren zum Herstellen eines eßbaren Materials, welches umfaßt: das Kultivieren eines ungiftigen Fungus in einem Nährstoffmedium, das fein geschnittenes Lignin-reiches grasartiges Material enthält, Zurückgewinnen des von der Kultivierung abgebauten Mediums und dann Entfernen des spezifischen Geruchs des abgebauten Mediums durch Erhitzen oder Fermentieren des Mediums.

2. Verfahren nach Anspruch 1, bei dem das grasartige Material aus Reisschalen, Gerstenschalen, Maiskolben, Bagasse, Buchweizenschalen, Sojabohnenschalen, Stengel oder Blätter von Gramineae und Mischungen davon ausgewählt ist.

3. Verfahren nach Anspruch 1 oder 2, bei dem das geschnittene grasartige Material eine Volumendichte von 0,15 bis 0,66 hat.

4. Verfahren nach einem der vorhergehenden Ansprüche, bei dem das Nahrungsmedium auch eine Nahrungsquelle, ausgewählt aus Reiskleie, Weizenkleie, Sojabohnen-Quarksediment, Reismehl, Weizenmehl, Sojamehl und Mischungen davon enthält.

5. Verfahren nach einem der vorhergehenden Ansprüche, bei dem das Gewichtsverhältnis des grasartigen Materials zu den Nährstoffquellen 3:2 bis 4:2 ist.

6. Verfahren nach einem der vorhergehenden Ansprüche, bei dem der ungiftige Fungus ein Speisepilz ist.

7. Verfahren nach einem der vorhergehenden Ansprüche, bei dem die Erwärmung bei einer Temperatur von nicht weniger als 70°C ausgeführt wird.

8. Verfahren nach Anspruch 7, bei dem die Erwärmung durch Dampf bei einer Temperatur von nicht weniger als 95°C ausgeführt wird.

9. Verfahren nach einem der Ansprüche 1 bis 6, bei dem die Fermentierung unter Verwendung eines ungiftigen Mikroorganismus, der aus Hefe, Fungi und Bakterien ausgewählt ist, ausgeführt wird.

10. Verfahren nach Anspruch 9, bei dem der Mikroorganismus eine Hefe, Lactobazillus, Aspergillus oder Bazillus natto ist.

11. Eßbares Material, welches ein abgebautes Medium aufweist, das durch Kultivieren eines ungiftigen Fungus in einem Nahrungsmedium, das fein geschnittenes Lignin-reiches grasartiges Material beinhaltet, erhalten wird, und bei dem der spezifische Geruch des abgebauten Mediums durch Erwärmen oder Fermentieren des Mediums entfernt worden ist.

## Revendications

1. Procédé de préparation d'une matière comestible qui comprend la culture d'un champignon non toxique dans un milieu nutritif contenant une matière herbacée riche en lignine coupée finement, la récupération du milieu digéré par la culture puis l'élimination de l'odeur spécifique du milieu digéré par chauffage ou fermentation du milieu.

2. Procédé selon la revendication 1, dans lequel la matière herbacée est choisie parmi des enveloppes de riz, des enveloppes d'orge, des rafles de maïs, de la bagasse, des enveloppes de sarrasin, des enveloppes de soja, les tiges ou les feuilles de graminées, et les mélanges de celles-ci.

3. Procédé selon la revendication 1 ou la revendication 2, dans lequel la matière herbacée coupée a une densité volumique de 0,15 à 0,66.

4. Procédé selon l'une quelconque des revendicntions précédentes, dans lequel le milieu nutritif contient également une source nutritive choisie parmi le son de riz, le son de blé, les lies de soja, la poudre de riz, la farine de blé, la poudre de soja et les mélanges de ceux-ci.

5. Procédé selon l'une quelconque des revendications précédentes, dans lequel le rapport pondéral de la matière herbacée aux sources nutritives est de 3:2 à 4:2.

6. Procédé selon l'une quelconque des revendications précédentes, dans lequel le champignon non toxique est un champignon comestible.

7. Procédé selon l'une quelconque des revendications précédentes, dans lequel ledit chauffage est effectué à une température non inférieure à 70°C.

8. Procédé selon la revendication 7, dans lequel ledit chauffage est effectué par la vapeur à une température non inférieure à 95°C.

9. Procédé selon l'une quelconque des revendications 1 à 6, dans lequel ladite fermentation est effectuée en utilisant un microorganisme non toxique choisi parmi les levures, les champignons et les bactéries.

10. Procédé selon la revendication 9, dans lequel le microorganisme est une levure, le *Lactobacillus*, *l'Aspergillus* ou le *Bacillus natto*.

11. Matière comestible qui comprend un milieu digéré obtenu par culture d'un champignon non toxique dans un milieu nutritif contenant une matière herbacée riche en lignine coupée finement, et dont on a éliminé l'odeur spécifique dudit milieu digéré par chauffage ou fermentation du milieu.
